# EUROPEAN PATENT APPLICATION

(11) **EP 3 473 705 A1**
(43) Date of publication of application: **24.04.2019**
(21) Application number: 17815338.3
(22) Date of filing: 19.06.2017
(51) Int. Cl.: C12N 5/071, A61L 27/36, A61L 27/60, C12N 5/0775

(54) **METHOD FOR PRODUCING ARTIFICIAL SKIN HAVING HAIR FOLLICLES, SEBACEOUS GLANDS, AND PORES**

(30) Priority: 21.06.2016 JP 2016122719
(71) Applicant: Organ Technologies, Inc., Minato-ku Tokyo 105-0001 (JP); Riken, Wako-shi, Saitama 351-0198 (JP)
(72) Inventor: TSUJI, Takashi, Wako-shi Saitama 351-0198 (JP); TOYOSHIMA, Koh-ei, Tokyo 105-0001 (JP); OGAWA, Miho, Tokyo 105-0001 (JP)
(74) Representative: Weickmann & Weickmann PartmbB
(86) International application number: PCT/JP2017/022479
(87) International publication number: WO 2017/221870

(57) **Abstract**

The object of the present invention is to provide an artificial skin having hair follicles, sebaceous glands, and hair pores. The present invention provides a method for manufacturing artificial skin having hair follicles, sebaceous glands, and hair pores, characterized in that it comprises each of the following steps:
(A) : a step of preparing an artificial skin having dermal and epidermal layers or an artificial skin having only a dermal layer; and
(B): a step of transplanting isolated hair follicles to the artificial skin prepared in step (A);
wherein said isolated hair follicle comprises a sebaceous gland, and said isolated hair follicle is transplanted to said artificial skin so that the surface of said dermal layer of said artificial skin is in alignment with the position of the hair pore portion of said isolated hair follicle.

## Description

### Technical Field

The present invention relates to a method for manufacturing artificial skin, as well as an artificial skin manufactured by said method.

### Background Art

In recent years, in light of animal protection, development of technology to abolish animal experiments and to perform drug effect and safety evaluation of pharmaceuticals and cosmetics with a method alternative to animal experiment has been promoted around Europe. Moreover, in recent years, due to the progress in the development of cell culture technology for artificially proliferating cells taken from a living subject, it has become possible to perform ex vivo culture while retaining the physiological functions of various organs or tissues. As a result, various test evaluation methods employing human-derived cultured cells have been developed, and by employing these alternative evaluation methods, it has also become possible to investigate how a candidate pharmaceutical substance influences the human body or what dosage would exert the desired or adverse effect. Further, development of a method for manufacturing artificial skin by combining cultured cells, cell scaffolding, and culture medium has been promoted, and triggered by the invention of a three-dimensional cultured skin having function and structure equivalent to natural skin by Dr. H. Green, the dermatology field has been the groundbreaker in the development of methods alternative to animal experiment. Thus far, development and productization of artificial skin, as well as methods alternative to animal experiment employing artificial skin in order to perform drug effect and safety evaluation of pharmaceuticals and cosmetics on skin without laboratory animals have also been proposed (e.g. Patent Literature 1) .

The skin configured by epidermal and dermal tissues and subcutaneous tissue is a massive organ that thoroughly covers the body surface and separates the inside of the body from the external world. The skin is distributed with various ectodermal organs such as hair follicles, sebaceous glands, and sweat glands, and the integumentary system (skin organ system) is configured by structural and functional cooperation of these. The integumentary system defends the body surface from various external invasions by growing out hair, adjusts body surface environment or excretes waste product by secreting sebum and sweat, and further assumes assorted physiological functions such as thermoregulation and sensory reception by cooperating also with other organ systems such as the circulatory system and the nervous system. Since various water-soluble and lipophilic components secreted onto the skin surface by skin appendage changes the substance permeability and moisture content of the epidermal layer which is a rigid keratinized stratified epithelium, association with allergic diseases or aesthetic changes involved in aging are suggested.

However, artificial skin proposed thus far simply comprises only the epidermal and dermal layers without skin appendage, or did not reproduce normal skin structure and functions even if it comprised skin appendage-like structures (such as a hair follicle-like structure). Because skin barrier function by sebum etc. does not exist in artificial skin without functional skin appendage, its use as an alternative to a laboratory animal is limited.

For example, Patent Literature 1 shows that by overlaying and culturing a collagen matrix layer that comprises constrictor cells (such as fibroblasts) and a collagen matrix layer that comprises cells that constitute tissue appendage (such as hair papilla cells), hair follicle-like structures may be produced in the culture. However, Patent Literature 1 merely shows that a structure that has similar external form of a hair follicle was produced, and artificial skin that reproduces normal structure and function of skin appendage is not shown.

### Citation List

### Patent Literature

[Patent Literature 1] Japanese Published Unexamined Patent Application Publication No. H10-136977

### Summary of the Invention

### Problems to be Solved by the Invention

The object of the present invention is to provide an artificial skin having hair follicles, sebaceous glands, and hair pores.

### Means for Solving the Problems

As a result of extensive investigation by the present inventors to manufacture artificial skin that may reproduce animal skin barrier function, we have succeeded in manufacturing artificial skin having functional skin appendage.

In other words, in one embodiment, the present invention relates to a method for manufacturing artificial skin having hair follicles, sebaceous glands, and hair pores, characterized in that it comprises each of the following steps:
(A) : a step of preparing an artificial skin having dermal and epidermal layers or an artificial skin having only a dermal layer; and
(B): a step of transplanting isolated hair follicles to the artificial skin prepared in step (A);
   wherein
   said isolated hair follicle comprises a sebaceous gland, and
   said isolated hair follicle is transplanted to said artificial skin so that the surface of said dermal layer of said artificial skin is in alignment with the position of the hair pore portion of said isolated hair follicle.

Moreover, in one embodiment, the method of the present invention is characterized in that artificial skin having only a dermal layer is used in said step (A), and the method comprises the following step after said step (B) : (C) : a step of forming an epidermal layer on said dermal layer of said artificial skin.

Moreover, in one embodiment, the method of the present invention is characterized in that the dermal layer of the artificial skin in said step (A) is formed by gelling a mixed solution comprising fibroblasts, collagen, and culture medium.

Moreover, in one embodiment, the method of the present invention is characterized in that the dermal layer of the artificial skin in said step (A) is formed by gelling a mixed solution comprising fibroblasts, collagen, and culture medium, and then further adding a mixed solution comprising collagen and culture medium, and allowing it to gel and repeating this at least once or more times.

Moreover, in one embodiment, the method of the present invention is characterized in that the epidermal layer of the "artificial skin having dermal and epidermal layers" in said step (A) is formed by applying a mixed solution comprising keratinized cells and culture medium to the surface of said dermal layer, and subjecting them to culture.

Moreover, in one embodiment, the method of the present invention is characterized in that said step (C) is a step of forming said epidermal layer by applying a mixed solution comprising keratinized cells and culture medium to the surface of said dermal layer of said artificial skin, and subjecting them to culture.

Moreover, in one embodiment, the method of the present invention is characterized in that said step (A) is:
(A): a step of preparing an artificial skin having a dermal layer, an epidermal layer, and a fat tissue layer.

Moreover, in one embodiment, the method of the present invention is characterized in that it comprises the following step after said step (A) and before said step (B):
(A'): a step of embedding fat progenitor cells at the site of artificial skin that isolated hair follicles are transplanted in step (B).

Moreover, in one embodiment, the method of the present invention is characterized in that said isolated hair follicle is a hair follicle isolated from animal skin.

Moreover, in one embodiment, the method of the present invention is characterized in that said isolated hair follicle is a hair follicle isolated from human skin.

Moreover, in one embodiment, the method of the present invention is characterized in that said isolated hair follicle is an artificially induced regenerated hair follicle.

Moreover, in one embodiment, the method of the present invention is characterized in that said regenerated hair follicle is a regenerated hair follicle manufactured by putting a first cell aggregate composed substantially of mesenchymal lineage cells and a second cell aggregate composed substantially of epithelial cells in close contact and subjecting them to culture inside a support.

Moreover, in one embodiment, the method of the present invention is characterized in that at least either one of said mesenchymal lineage cells or said epithelial cells is obtained by inducing undifferentiated cells.

Moreover, in one embodiment, the method of the present invention is characterized in that at least either one of said mesenchymal lineage cells or said epithelial cells is singled cells derived from animal hair follicles.

In another embodiment, the present invention relates to an artificial skin having hair follicles and sebaceous glands, characterized in that it further has hair pores.

Moreover, in one embodiment, the present invention relates to an artificial skin comprising:
a dermal layer consisting of a gel comprising fibroblasts, collagen, and culture medium,
an epidermal layer that is formed on the surface of said dermal layer and substantially consists of keratinized cells, and
a hair follicle having a sebaceous gland, wherein
said hair follicle penetrates said epidermal layer and is buried into said dermal layer, and
the hair pore portion of said hair follicle is connected to said epidermal layer.

Moreover, in one embodiment, the artificial skin of the present invention is characterized in that it further has a fat tissue layer in the lower part of said dermal layer.

Moreover, in one embodiment, the artificial skin of the present invention is characterized in that said hair follicle is covered with fat progenitor cells in said dermal layer.

Moreover, in one embodiment, the present invention is characterized in that it is an artificial skin manufactured by the method of the present invention for manufacturing artificial skin described above.

An invention of any combination of one or more characteristics of the present invention described above is also encompassed by the scope of the present invention.

### Effects of the Invention

According to the method of the present invention, an artificial skin that has functional hair follicles, sebaceous glands, and hair pores, and reproduces normal skin barrier function can be manufactured. Accordingly, artificial skin useful for drug effect and safety evaluation of pharmaceuticals and cosmetics (in particular, drug effect and safety evaluation of pharmaceuticals and cosmetics related to hair) can be provided.

### Brief Description of the Drawings

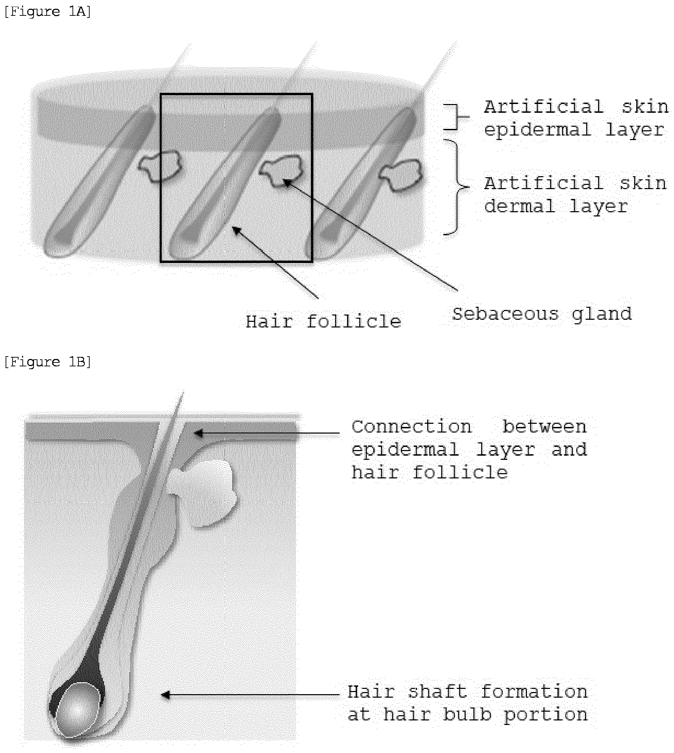

Figure 1 is a schematic diagram of artificial skin having hair follicles and sebaceous glands in which these and the epidermal layer are linked via the hair pore portion, manufactured by the method of the present invention. Figure 1A is a three-dimensional schematic diagram of artificial skin that incorporated skin appendage such as hair follicle. Figure 1B shows a cross-sectional schematic diagram including a hair follicle in the area shown in the rectangle in Figure 1A. The opening for hair follicle and sebaceous gland of the artificial skin is continuous with the keratinized epidermal layer of the artificial skin, and hair is erupted from its surface and sebum is secreted to the body surface.

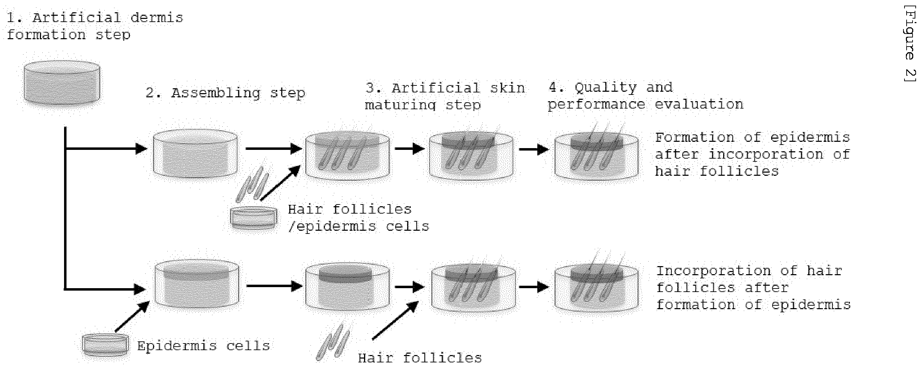

Figure 2 shows the two embodiments of the method of the present invention for manufacturing artificial skin. The method of the present invention is generally classified into two types, a method of forming the epidermis after incorporating hair follicles and a method of incorporating hair follicles after formation of epidermal layer, depending on the timing of the formation of the keratinized epidermal layer of the artificial skin and the incorporation of hair follicles comprising sebaceous glands.

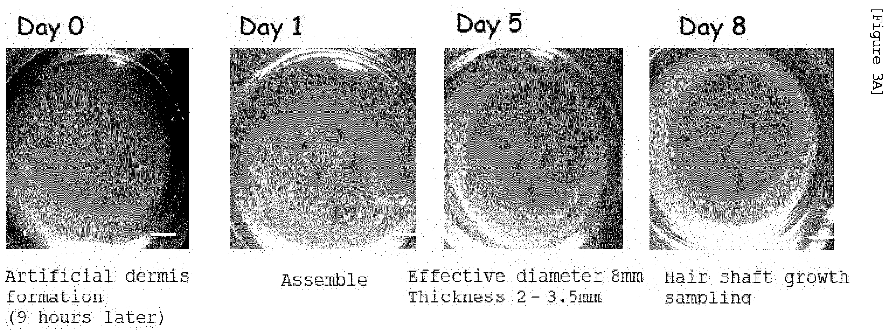

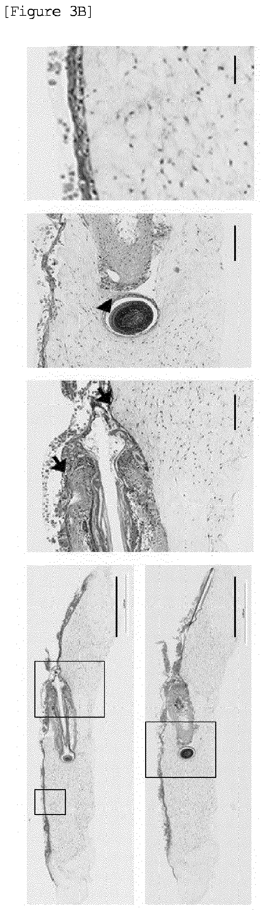

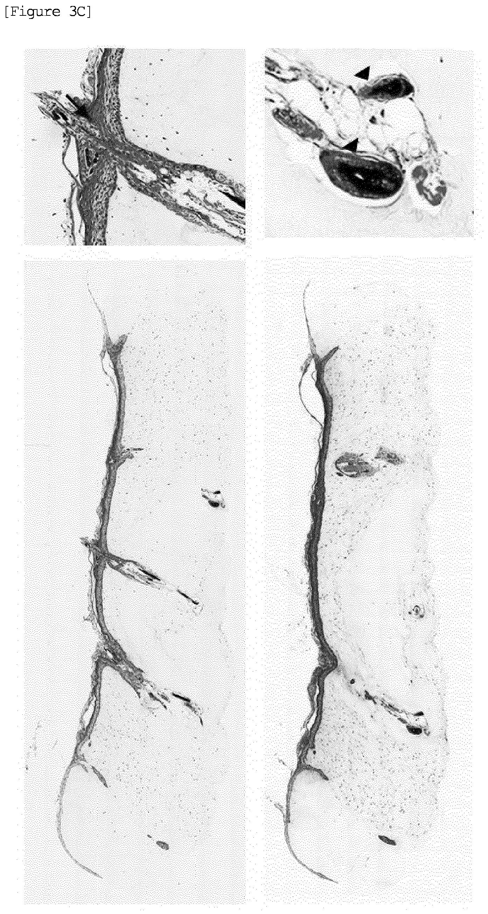

Figure 3 shows the result of transplanting isolated hair follicles to artificial skin having only a dermal layer, and then forming the epidermal layer and subjecting to culture according to one embodiment of the method of the present invention. Figure 3A shows photographs of formation of artificial skin having only a dermal layer (cell-containing collagen gel) on Day 0, incorporation of adult mouse whisker hair follicles in the artificial dermal layer on Day 1, performing overlaid seeding of cultured keratinocytes on the artificial dermis to carry out formation of the epidermal layer within the same day, and carrying out observation until Day 8. Figure 3B shows the tissue images of artificial skin when mouse whisker hair follicles were employed as the hair follicles to be transplanted in the procedures of Figure 3A. Low-power magnified images of serial sections of a tissue comprising hair follicles incorporated in the artificial skin are shown in the top and bottom left photographs, and magnified photographs of the areas shown with rectangles in top left and bottom left are shown in a - c. The magnified photograph areas are shown with symbols a - c in the low power magnifications. The continuous portion of the epithelium of the hair pore portion of the hair follicle and the keratinized epidermal layer of the artificial skin is shown with an arrow in a, and the hair bulb portion is shown with an arrowhead in b. Figure 3C shows tissue images of creating serial sections from artificial skin after incorporating mouse body hair follicles, subjecting to culture for seven days, and observation in the procedures of Figure 3A, as top and bottom photographs. The photographs on the left are low-power magnified images, and magnified images of areas shown with rectangles are each shown in the top and bottom photographs on the right. The hair follicles incorporated in the artificial skin and the keratinized epidermal layer of the artificial skin are connected via the hair pore portion in the arrowed area in the magnified image. Moreover, the hair bulb portion shown with arrowhead in the HE image of the hair bulb portion in the bottom right photograph is histologically healthy.

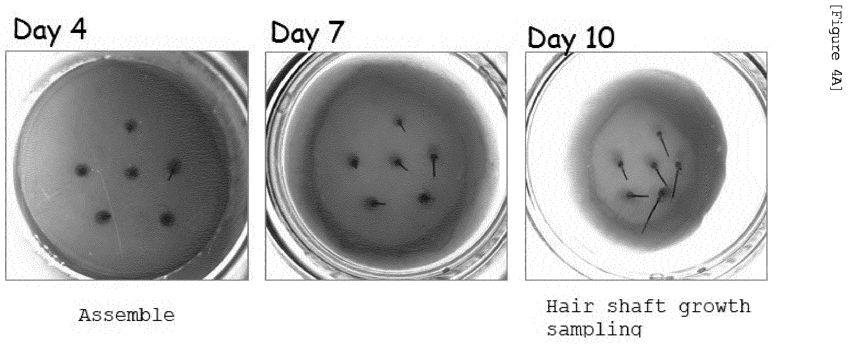

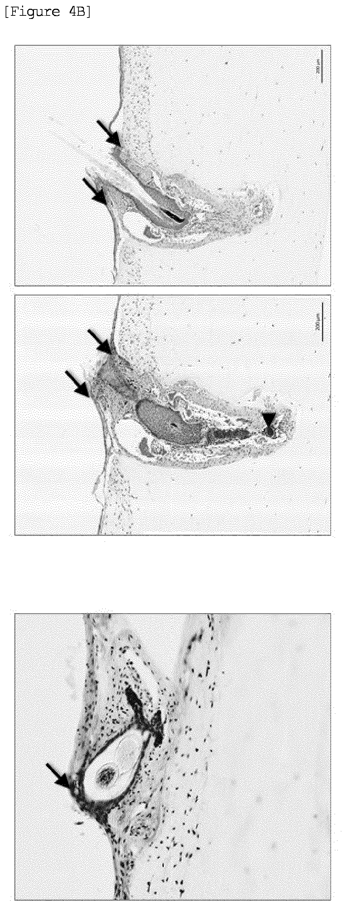

Figure 4 shows the result of transplanting isolated hair follicles to artificial skin having dermal and epidermal layers and subjecting to culture according to one embodiment of the method of the present invention. Figure 4A shows photographs of formation of artificial skin having dermal and epidermal layers (cell-containing collagen gel) on Day 0, transplantation of hair follicles (mouse whisker hair follicles) on Day 4, and carrying out observation until Day 10. Figure 4B shows the tissue images of artificial skin (H&E staining) after hair follicle transplantation. It is shown that the transplanted hair pore portion of the hair follicle and the epidermal layer of the artificial skin are connected in the arrowed area, hair matrix cells and hair papilla cells are surviving in the arrowhead area, and the hair bulb portion is histologically healthy.

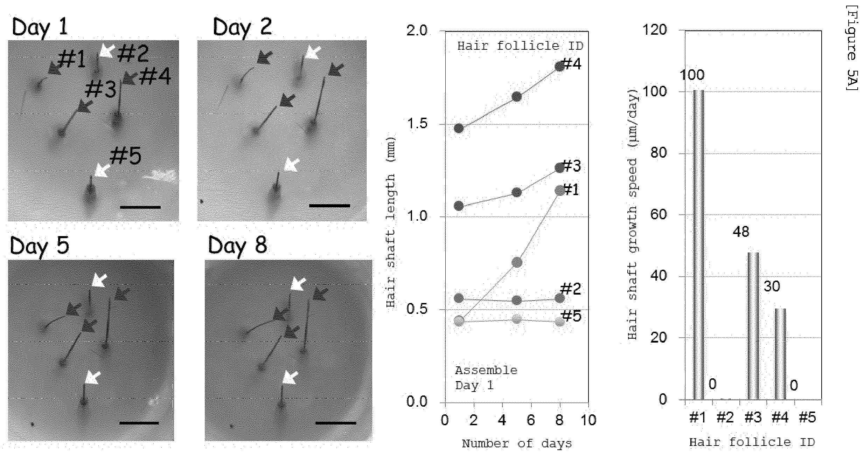

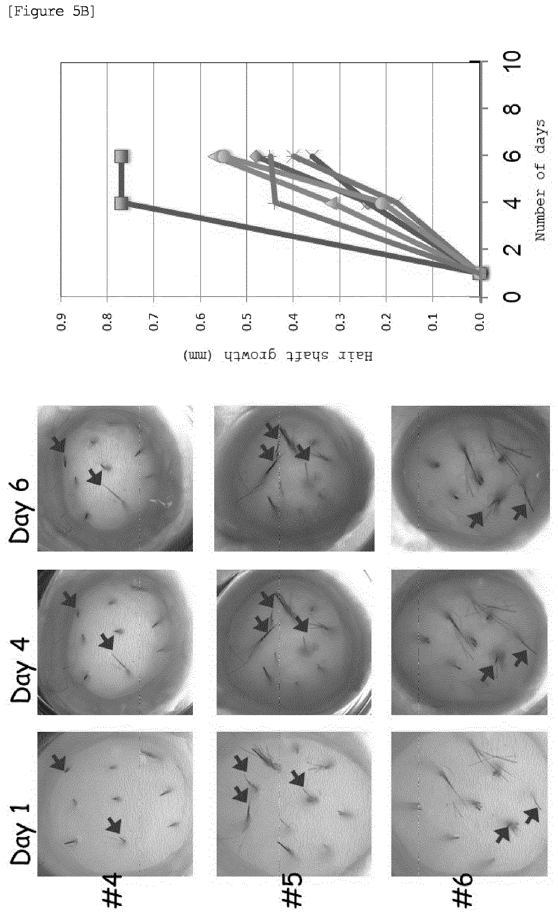

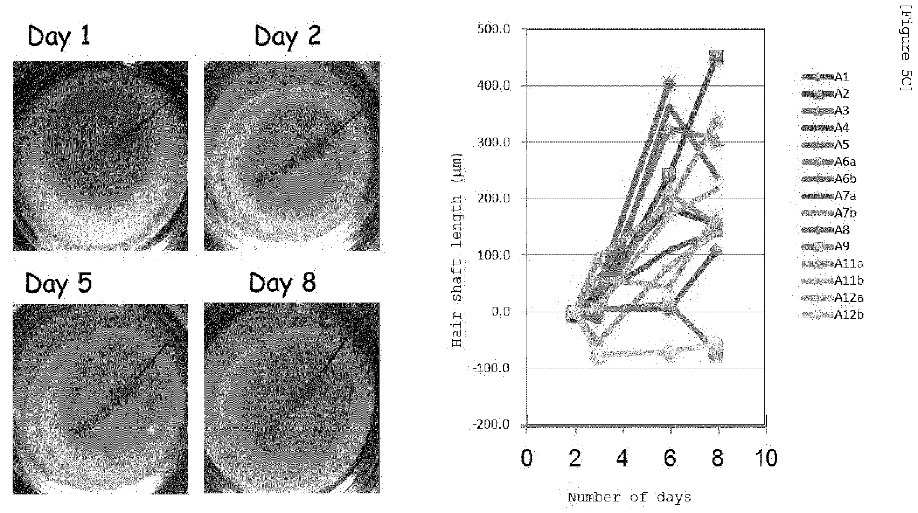

Figure 5 shows the result of observing hair shafts grown from hair follicles incorporated in the artificial skin over time and measuring hair shaft length by image analysis. Figure 5A is the observation result of hair shaft growth when the epidermal layer was formed after transplanting adult mouse whisker hair follicles to artificial skin. Multiple hair follicles incorporated to artificial skin were each discriminated, and hair shaft growth of each was tracked over time. Hair shafts that grew are shown with red arrows, and hair shafts that did not grow are shown with white arrows. Figure 5B is the observation result of hair shaft growth when the epidermal layer was formed after transplanting adult mouse body hair follicles to artificial skin. Similarly to observation of mouse whiskers over time, hair follicles incorporated in the artificial skin were discriminated, and hair shaft length of hair follicles that showed hair growth (red arrows) were measured. Figure 5C is the observation result of hair shaft growth when the epidermal layer was formed after human head hair follicles were transplanted to artificial skin. Similarly to mouse hair follicles, hair shaft length was measured and plotted.

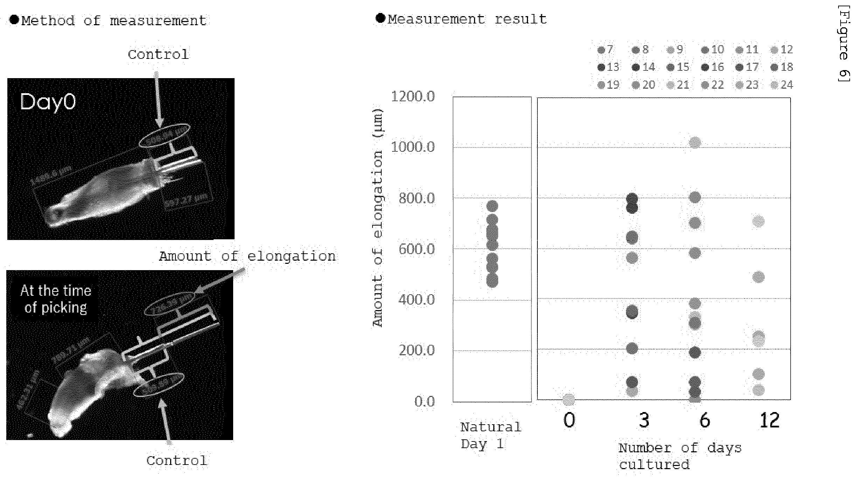

Figure 6 shows the result of observing hair shaft growth after transplanting hair follicles to artificial skin having dermal and epidermal layers. Stereomicroscopic photographs of hair shafts were taken before transplantation of hair follicles, the lengths of club hair and anagen hair shaft were measured from the topmost end of the hair bulb portion, hair shafts and hair follicles were resected from artificial skin on Days 3, 6, and 12 after transplantation to artificial skin, and stereomicroscopic photographs were similarly taken to measure the amount of hair shaft growth. With the length of club hair as internal standard, the elongated amount of anagen hair was measured and plotted.

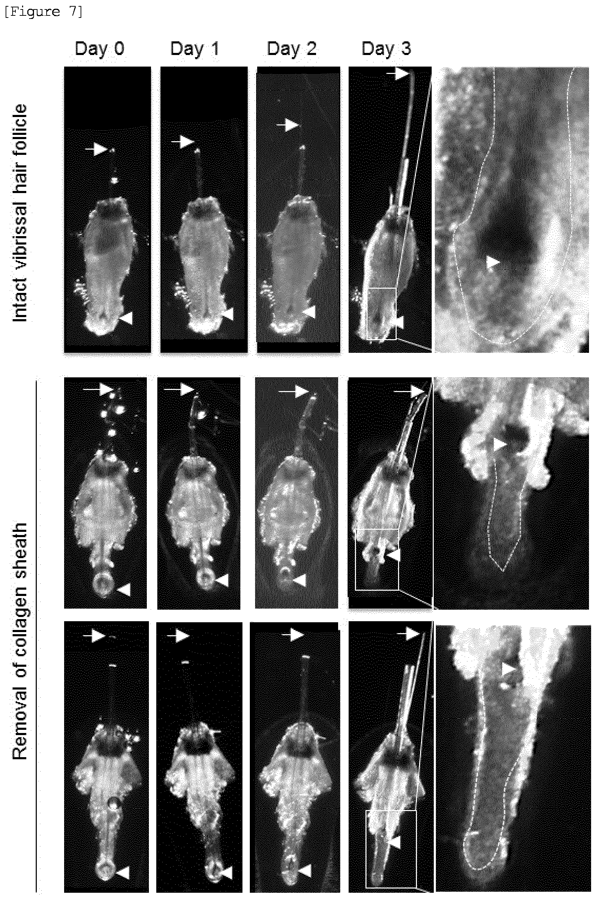

Figure 7 shows the change over time of mouse whisker hair follicles in organ culture. Mouse whiskers in anagen were harvested to produce intact hair follicles comprising collagen sheath (top row) and hair follicles with collagen sheath in the variable region removed (bottom row). Hair follicles for each condition were adhered to the bottom of a 6 cm plastic culture dish with a thin layer collagen gel, subjected to immersion culture in DMEM/F12 (1:1) comprising 10% FBS under 5% CO2 environment until Day 3. This was followed up daily with a stereomicroscope from the start of culture (Day 0). The arrows show hair shaft tips, and the arrowheads show the positions of the hair bulb portion. The right column shows magnified images of the hair bulb portion on culture Day 3. The dotted line shows the mesenchymal tissue boundary of the hair bulb portion. The off-position between the positions of the hair bulb portion and the mesenchymal tissue boundary of the hair bulb portion indicates that it is catagen or telogen.

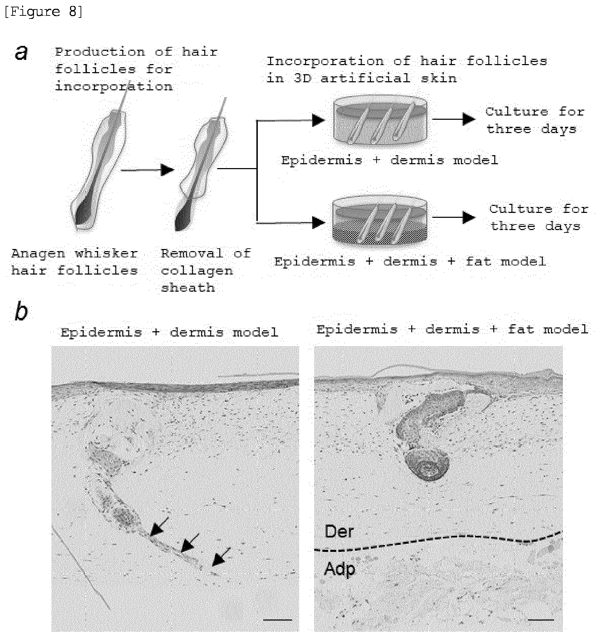

Figure 8 shows the comparison between an artificial skin model with only the epidermis and dermis and an artificial skin model with epidermis, dermis, and fat tissue layer. Figure 8a shows the flow of this experiment. Mouse whiskers in anagen were harvested to produce hair follicles with collagen sheath in the variable region removed. These were incorporated in the epidermis + dermis model or the epidermis + dermis + fat tissue models of artificial skin produced as 6-well formats, and subjected to organ culture for three days. The organ culture condition was DMEM/F12 (1:1) comprising 10% FBS, under 5% CO2 environment, and carried out by semi-gas phase culture. Figure 8b shows the tissue images on organ culture Day 3. Hair follicles incorporated in the epidermis + dermis model (left figure) was a linear hair follicle mesenchyme image (arrows) indicating catagen, whereas hair follicles incorporated in the epidermis + dermis + fat tissue model (right figure) were indicated to be anagen hair bulb portions. The dotted line in the right photograph shows the boundary between the dermis (Der) and fat tissue (Adp) .

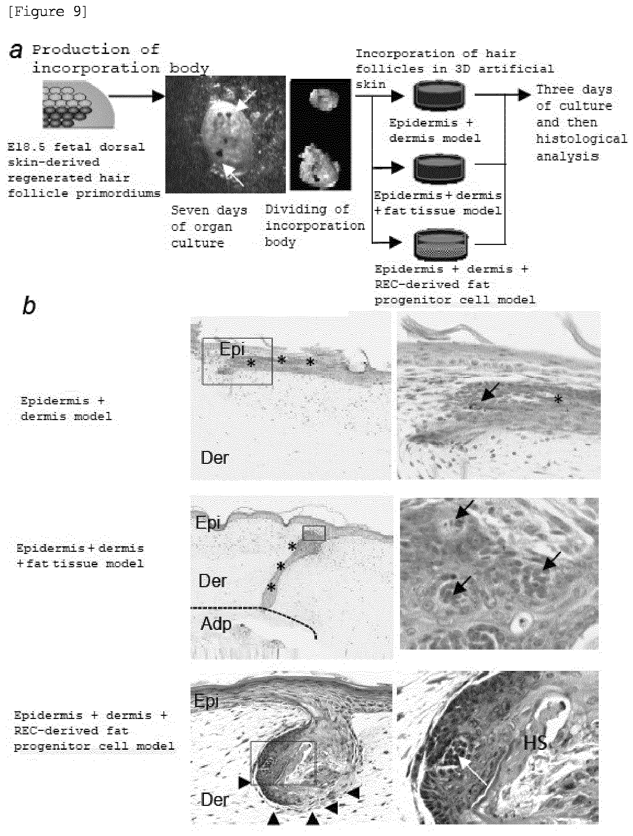

Figure 9 shows the result of producing the artificial skin of the present invention with artificially produced regenerated hair follicles.
Figure 9a shows the flow of this experiment. In Figure 9b, the top row shows the result of the "epidermis + dermis model," the middle row shows the "epidermis + dermis + fat tissue model, " and the bottom row shows the "epidermis + dermis + REC-derived fat progenitor cell model." Magnification of the rectangular area shown in the left column of Figure 9b, the low-power magnified view, is shown in the right column. In any of the models, it was shown that the epithelium tissue of the transplanted hair follicles is connected to the epidermal layer of the artificial skin.

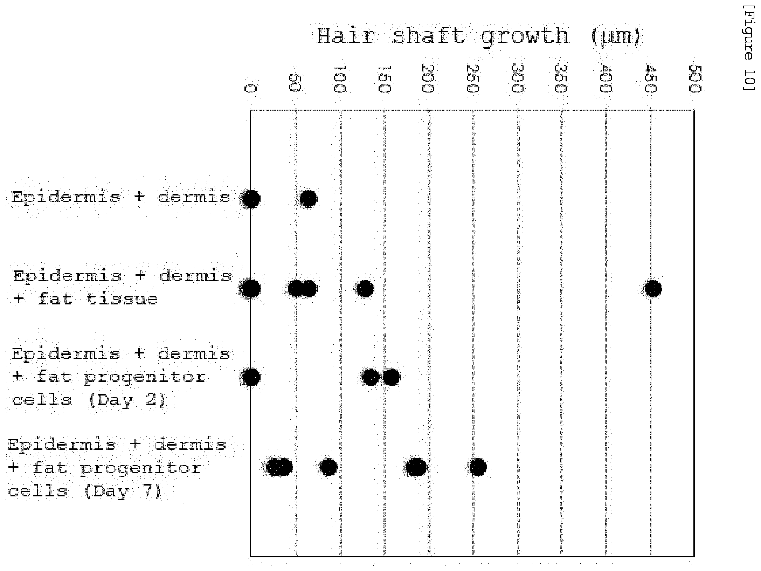

Figure 10 shows the result of comparing hair shaft growth in each of the "epidermis + dermis model," the "epidermis + dermis + fat tissue model," and the "epidermis + dermis + REC-derived fat progenitor cell model."

### Description of Embodiments

The present invention relates to an artificial skin having hair follicles, sebaceous glands, and hair pores, as well as a manufacturing method thereof. The artificial skin manufactured by the method of the present invention is characterized in that it has a hair follicle having a sebaceous gland, said hair follicle penetrates the epidermal layer and is buried into the dermal layer, and the hair pore portion of said hair follicle is connected to said epidermal layer.

In a living subject, a hair follicle means skin appendage that produces a hair having structures such as hair papilla, hair matrix, root sheath, hair fiber, hair bulge, arrector pili muscle, sebaceous gland, and hair follicle infundibular part (so-called hair pore). However, a "hair follicle" as used herein is not limited to those comprising all of these structures, but broadly comprises those that comprise at least a part of these structures and maintain structures that have the function to produce hair.

An "artificial skin" as used herein refers to an artificial dermatoid structure that has at least a dermal layer, preferably to those that further has an epidermal layer. A "dermal layer" as used herein means a structure composed mainly of collagen or fibroblasts, and an "epidermal layer" means a structure composed mainly of keratinized cells (keratinocytes). The artificial skin employed in the present invention (artificial skin before transplantation of isolated hair follicles) may be prepared before transplantation of hair follicles, or a commercially available artificial skin that has epidermal and dermal layers may be employed.

The method for manufacturing the artificial skin employed in the present invention (artificial skin before transplantation of isolated hair follicles) is not limited, and the dermal layer can be produced by for example gelling a mixed solution comprising fibroblasts, collagen, and culture medium. Preferably, after gelling a mixed solution comprising fibroblasts, collagen, and culture medium, a mixed solution comprising collagen and culture medium is further added and allowed to gel, and this may be repeated at least once or more times to form a dermal layer. The origin of the fibroblasts employed for producing the dermal layer of the artificial skin is not limited, and for example, fibroblasts derived from human, monkey, pig, cow, horse, dog, cat, mouse, rat can be employed. Moreover, the fibroblasts employed for producing the dermal layer of the artificial skin may be derived from any site of a living subject, and for example, fibroblasts derived from neonatal, fetal, or adult buttock, scalp, palm, sole, and preputium can be employed.

Moreover, the epidermal layer of the artificial skin of the present invention can be formed by for example applying a mixed solution comprising keratinized cells and culture medium to the surface of the dermal layer produced by the method described above and subjecting them to culture. The origin of the keratinized cells employed for producing the epidermal layer of the artificial skin is not limited, and for example, keratinized cells derived from human, monkey, pig, cow, horse, dog, cat, mouse, rat can be employed. Moreover, the keratinized cells employed for producing the epidermal layer of the artificial skin may be derived from any site of a living subject, and for example, keratinized cells derived from neonatal, fetal, or adult buttock, scalp, palm, sole, and preputium can be employed.

Moreover, the artificial skin of the present invention may further have a fat tissue layer under the dermal layer. A "fat tissue layer" as used herein is not particularly limited as long as is comprises fat cells and is a structure have a composition that allows cell culture, and for example may be a collagen gel comprising fat tissue.

Moreover, although hair follicles penetrate the epidermal layer and be buried in the dermal layer in the artificial skin of the present invention, it may be configured so that said hair follicles are covered with fat progenitor cells in said dermal layer. The method for producing artificial skin having such a configuration is not limited, and the desired configuration can be obtained by for example embedding fat progenitor cells at the hair follicle transplantation site before transplanting the hair follicles to artificial skin consisting of epidermal and dermal layers, and then transplanting the hair follicles. Further, the desired configuration can also be obtained for example by adhering fat progenitor cells around the hair follicles for transplantation, and then transplanting the hair follicles to artificial skin consisting of epidermal and dermal layers.

The origin of the fat progenitor cells employed in the present invention is not particularly limited, and it may be commercially available fat progenitor cells, may be fat progenitor cells induced by a well-known method from mesenchymal lineage stem cells that are commercially available or isolated from a living subject, or may be fat progenitor cells directly isolated from a tissue of a living subject.

The isolated hair follicles employed in the present invention may be isolated hair follicles harvested from animal skin. In this case, the type of animal for harvesting the hair follicles is not limited, and for example, isolated hair follicles derived from human, monkey, pig, cow, horse, dog, cat, mouse, rat can be employed. The method for trimming the hair follicles harvested from animal skin is not limited, and for example, the isolated hair follicles employed for the present invention can be obtained by isolating hair follicles from harvested skin so as not to damage the sebaceous gland.

Moreover, artificially induced hair follicles can also be employed as the isolated hair follicles employed in the present invention. The method for manufacturing the artificially induced hair follicles is not limited, and for example, artificially induced hair follicles can be employed using the methods described in WO2012/108069 or WO2012/115079. Specifically, regenerated hair follicle primordium-derived hair follicles manufactured by putting a first cell aggregate composed substantially of mesenchymal lineage cells and a second cell aggregate composed substantially of epithelial cells in close contact and culturing them inside a support can be employed in the present invention. In this case, at least either one of said mesenchymal lineage cells or said epithelial cells may be mesenchymal lineage cells or epithelial cells obtained by inducing undifferentiated cells (such as iPS cells, ES cells, various tissue stem cells, or various progenitor cells). Moreover, at least either one of said mesenchymal lineage cells or said epithelial cells may be mesenchymal lineage cells or epithelial cells obtained from singled cells derived from animal hair follicles. Moreover, regenerated hair follicle primordiums obtained by the above method may be transplanted to the skin of a living subject, reharvested once the hair follicles have grown to some extent, and employed for the present invention.

Moreover, for example, artificially induced hair follicles can be employed for the present invention by using the method described in WO2016/039279. Specifically, pluripotent stem cell-derived embryoids are stimulated with a biologically active substance that may activate the Wnt pathway, and then said embryoids are bound to a scaffolding, and said bound object is transplanted to a living animal body. A teratoma that contains skin appendage such as hair follicle or sebaceous gland at high frequency is then formed at said transplantation site, and hair follicles harvested from said teratoma can be employed for the present invention.

In the method of the present invention for manufacturing artificial skin, isolated hair follicles are transplanted to artificial skin so that the surface of the dermal layer of the artificial skin and the position of the hair pore portion of said isolated hair follicle are substantially in alignment. By virtue of this step, the hair pore portion of the hair follicle and the epidermal layer of the artificial skin are connected after hair follicle transplantation, and the hair follicles and sebaceous glands will become functional as skin appendages. The hair pore portion of the hair follicle and the epidermal layer of the artificial skin are "connected" (or the hair pore portion of the hair follicle and the epidermal layer of the artificial skin are "continuous") as used herein means that the hair pore portion of the hair follicle and the epidermal layer of the artificial skin are at least partially histologically bound.

The terms used herein, unless particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### Example 1: Manufacture of artificial skin having hair follicles, sebaceous glands, and hair pores

The schematic diagram of the artificial skin manufactured by the method of the present invention is shown in Figure 1. Moreover, the procedures for the two embodiments of the method of the present invention for manufacturing artificial skin carried out in this Example are shown in Figure 2.

### 1. Materials and Methods

### (1) Laboratory animals

B57/B6 mice were purchased from Shimizu Laboratory Supplies, Co., Ltd. (Tokyo, Japan). Management and operation of the mice was in accordance with NIH's Laboratory Animal Guideline. All experiments were reviewed by RIKEN's animal experiment committee and carried out with its approval.

### (2) Human materials

In this research, human materials were harvested and donated at a joint research medical institution in line with the gist of the "Declaration of Helsinki" (amended in 1975, Tokyo), after consult and approval from the research ethics review committee at RIKEN and the donor medical institution, in compliance with "Ethical Guidelines for Medical and Health Research Involving Human Subjects" (Public Notice of the Ministry of Education, Culture, Sports, Science and Technology and Ministry of Health, Labour and Welfare No. 3 of 2014) and related regulations, and with informed consent from the donors, and used at a predetermined facility at RIKEN Center for Developmental Biology. Human materials in this research refer to scalp tissue donated at medical institutions in Japan, as well as hair follicles and surrounding tissue separated therefrom, and do not comprise purchased cultured human-derived cells.

### (3) Cell culture

Normal human neonatal preputium fibroblasts (HDFn) and normal human neonatal preputium epidermal keratinized cells (HEKn) were purchased as frozen cells after first passage from KURABO. HDFn and HEKn cells were rapidly thawed in a warm bath at 37°C, and then washed with 10% fetal bovine serum and Dulbecco modified Eagle's medium (DMEM 10) comprising 50 units/ml of penicillin and 50 µg/ml of streptomycin, seeded at a cell density of 2,500 cells/cm² in a culture plastic dish filled with DMEM 10 medium for HDFn cells and HuMedia-KG2 medium (KURABO) for HEKn cells, and these were called second passage cells. Passage culture was according to conventional means, digested with a solution of D-PBS (-) (Nacalai Tesque) supplemented with 0.25% Trypsin-1 mM EDTA (Invitrogen), dispersed, and then the cell density was adjusted and subcultured under medium conditions set for each cell. Normal human cells were subcultured to the third passage and subjected to artificial skin production, or set as frozen stocks and employed for artificial skin production after thawing as fourth passage cells. When used as frozen stocks, both were maintained in culture until 60 - 80% confluent, and used at a state of 80% or higher confluency for artificial skin production.

### (4) Production of fibroblast-containing collagen gel

HDFn cells subcultured in (3) were single-celled by digestion by trypsin EDTA solution. Subsequently, a neutral collagen solution comprising final concentrations of 3.8 mg/ml collagen (I-AC 5 mg/mL, KOKEN), 1 x DMEM (SIGMA), 10 mM NaHCO3 (Wako Pure Chemical Corporation), 10 mM HEPES buffer (titered and adjusted to pH 7.4, Wako Pure Chemical Corporation), and 5% fetal bovine serum was produced, and HDFn cells pelleted by centrifugation was dispersed while gently stirring at ice temperature condition. As an additive to neutralize an acidic collagen solution to obtain a cell survival environment, 10 x concentration DMEM, 1M NaHCO3, and 1M HEPES buffer were produced, and stored as stock under refrigeration at 4°C. HDFn cells dispersed in the neutral collagen solution were dispensed in a 12-well cell culture insert (0.4 µm/high density pore) at 400 µl each, left standing to gel under 37°C, 5% CO2, and 100% humidity condition for 30 minutes to form an artificial dermal layer with a thickness of 3 - 4 mm. In order to make the surface of the fibroblast-containing collagen gel horizontal with the filter surface of the top of the cell culture insert, as well as to allow uniform gelling progress, preparation of the collagen solution and cell dispersion were performed on ice, and the prepared collagen solution was stored buried in ice until dispensed into the cell culture insert. The cell culture insert having formed an artificial dermal layer by gelling of the cell-containing collagen solution was set in a 12-well culture plate filled with DMEM 10 medium comprising 10 ng/ml of FGF2 (Wako Pure Chemical Corporation) and cultured overnight. The following culture conditions were all 37°C, 5% CO2, and 100% humidity condition.

After culturing human fibroblast-containing collagen gel overnight, gel shrinking by microfibril formation was visually confirmed. Then, 100 µl of collagen solution comprising final concentrations of 3.8 mg/ml of cow-derived acidic natural collagen 1 solution (I-AC 5 mg/mL, KOKEN), 1 x DMEM (SIGMA), 10 mM NaHCO3 (Wako Pure Chemical Corporation), 10 mM HEPES buffer (pH 7.4), and 5% fetal bovine serum was placed in a new cell culture insert, and the shrunk gel was transferred with tweezers. This was then left standing to gel under 37°C, 5% CO2, and 100% humidity condition for 30 minutes, and the periphery gap was filled with collagen gel containing no cells to prevent the medium component of the 12-well plate from flowing into the cell culture insert. A similar treatment was carried out at a frequency of once a day for three days after gel formation. A macrophotograph of the fibroblast-containing collagen gel was taken at each treatment, and it was measured that shrinking rapidly progressed until Day 3 and then comes to be an almost stationary state, and it was also confirmed that the fibroblast-containing collagen gel was produced to function physiologically appropriately.

### 2. Production of artificial skin by forming epidermis after incorporating hair follicles into dermal layer (Figure 3)

Artificial skin was produced by incorporating hair follicles in the fibroblast-containing collagen gel produced by the methods of the above (1) - (4) and then overlaying the epidermal layer.

The specific method is as follows. First, on Day 1 after the formation of fibroblast-containing collagen gel, an incision for hair follicle incorporation was made to said collagen gel. With an ophthalmic microscalpel (straight knife Straight 22.5°, MANI), a slit about 1.2 times the width of the hair follicle was formed onto the gel surface. The slit was made with a depth to the limit of not damaging the filter surface at the bottom of the cell culture insert, with an inclination of about 30 degrees. Into this slit, a whisker hair follicle, a body hair follicle of trunk dorsal skin, or a human head hair follicle (all of which are isolated hair follicles) was inserted from the surface side. Only the hair shaft extending from the hair follicle to be inserted was grabbed with tweezers, and the hair bulb portion or the hair pore portion remained untouched. Upon insertion, the hair follicle was immersed in neutral collagen solution containing no cells and then incorporated into the slit. The depth of the transplanted hair follicle was adjusted so that the hair pore portion of the hair follicle and the surface of the fibroblast-containing collagen gel are nearly in alignment.

Then, the epidermal layer was formed on the surface of the fibroblast-containing collagen gel by a method similar to "3. Production of artificial skin by incorporating hair follicles after formation of dermal and epidermal layers" described below, and air culture was performed.

For the hair follicles to be incorporated into the artificial skin, whiskers and body hair of trunk dorsal skin from B57/B6 mice at 5 - 7 days old after birth, as well as human head hair follicles in anagen VI phase that are growing hair shafts of which the hair type can be identified from the body surface were employed. Moreover, mouse whiskers and dorsal skin body hair as well as human scalp hair follicles in which club hair still remains were separated and processed with a stereomicroscope based on skin transplantation technology of hair follicle organs described in each prior literature (Toyoshima et al. (NATURE COMMUNICATIONS, 3:784, DOI:10.1038/ncomms1784,2012), Asakawa et al. (SCIENTIFIC REPORTS, 2:424, DOI:10.1038/srep00424,2012), Under et al. (Hair Transplantation 5th edn (2010))). For mouse whiskers, the dermal tissue and the collagen sheath located above the narrow portion of the hair follicle as well as the skin epidermal layer were excised with a surgical scalpel so as to retain the collagen sheath and not damage the sebaceous gland and the outer root sheath. Body hair was cut up from the skin and separated as hair groups consisting of 10 - 15 complete hair follicles so as to comprise the skin epidermal layer, the dermal layer, and the subcutaneous fat layer, and be horizontal to the hair follicles. Human hair follicles were separated to obtain a state of hair groups consisting of one or two hair follicles, and the dermal tissue above the hair follicle and the skin epidermal layer were trimmed similarly to whisker.

### 3. Production of artificial skin by incorporating hair follicles after formation of dermal and epidermal layers (Figure 4)

Artificial skin was produced by forming an epidermal layer on the surface of the fibroblast-containing collagen gel produced by the methods of the above (1) - (4) and then incorporating hair follicles.

The specific method is as follows. HEKn cells proliferated to 60% - 80% confluency were single-celled by trypsin digestion, and suspended in HuMedia-KG2 medium to 2 x 10⁶ cells/ml. Five hundred microliters of cell suspension per well was seeded overlaid on the fibroblast-containing collagen gel produced in (4) and cultured to form a keratinized epidermal layer on the fibroblast-containing collagen gel. The overlaid HEKn cells were cultured in HuMedia-KG2 medium or DMEM 10 medium until Day 4 after seeding, and total medium exchange was performed on Days 1, 2, and 3 after HEKn cell seeding with the same medium condition as the overlaid seeding. The operation described above was performed after replenishing the collagen gel following the shrinking of the fibroblast-containing collagen gel. The amount of DMEM 10 medium comprising 10 ng/ml of FGF2 in the well was adjusted so as to be the same with the liquid surface of the HuMedia-KG2 medium in the cell culture insert. By removing the medium in the cell culture insert on Day 4 after overlay of HEKn cells, air culture, which is a culture condition in which the epidermal layer is directly exposed to atmospheric oxygen partial pressure condition, was initiated.

Next, hair follicle incorporation was performed on the fibroblast-containing collagen gel having an epidermal layer formed. HEKn cells were overlaid on the HDFn cell-containing collagen gel, hair follicle incorporation was performed on Day 3, and air culture was initiated on Day 4. In this Example, isolated mouse whiskers prepared in the method described in "2. Production of artificial skin by forming epidermis after incorporating hair follicles into dermal layer" were employed. Incorporation of hair follicles in the artificial skin was performed similarly to the method described in "2. Production of artificial skin by forming epidermis after incorporating hair follicles into dermal layer" by forming slits that penetrate both the epidermal layer and the cell-containing collagen gel layer. In order to prevent detachment of the overlaid epidermal layer and the cell-containing collagen gel layer upon slit formation, first incisions in the epidermal layer were formed by making shallow movements with an ophthalmic microscalpel in the horizontal direction, and then incisions in the dermal layer were made.

### 4. Functional evaluation of hair follicles incorporated in artificial skin

### 4-1. Interepithelial connection

Ectodermal organs such as hair follicles or sweat glands function as skin organ systems (integumentary systems) by linking with the skin epidermal layer via openings such as hair pores and ducts. In other words, in order for the sebaceous glands incorporated together with the hair follicles into the artificial skin to be functional, it is essential that the hair pore portion of the incorporated hair follicle and the epidermal layer of the artificial skin are continuous.

The medium was removed on Day 4 and Day 7 after incorporating hair follicles, and formalin fixative (SuperFix, KURABO) was added into the cell culture insert and the plate well at 1 ml and 2 ml, respectively, to allow tissue fixation. Tissue fixation was performed at room temperature environment for 6-12 hours, transferred to PBS (-) at room temperature at the end of fixation, and transverse section vertical to the epidermal layer comprising the midline of the artificial skin that incorporated hair follicles and the hair pores and the hair growth direction was cut out to produce paraffin-embedded blocks. Serial sections at a thickness of 10 µm were then created according to a conventional method, H&E staining was performed, the positions of continuity between the hair follicles and the epidermal layer as well as the hair bulb portion connected thereto were tracked.

As shown in Figures 3B and 3C, it was shown in the artificial skin having formed the epidermis after incorporating hair follicles into the dermal layer that the hair follicle outer root sheath and the epidermal layer of the artificial skin are linked in the upper hair pore region of the hair follicles incorporated in the artificial skin.

Moreover, as shown in Figure 4B, it was shown that the hair follicle outer root sheath and the epidermal layer of the artificial skin are also linked in the upper hair pore region of the hair follicles incorporated in the artificial skin in artificial skin having incorporated hair follicles after formation of dermal and epidermal layers.

From these results, it was shown that the hair follicles in the artificial skin produced by the method of the present invention are linked with the artificial skin epidermal layer via hair pores and has functional skin appendage.

### 4-2. Histological evaluation

Using the H&E staining images of Day 4 and Day 7 after incorporating hair follicles, non-physiological degeneration of hair matrix cells of the hair bulb portion and hair papilla cells, or nuclear morphology accompanying cell death or change in cytoplasmic stainability, as well as cell arrangement specific to each were histologically analyzed, and compared to that of natural hair follicle tissue.

By histologically analyzing whether cells constituting the hair bulb portion of hair follicles incorporated in artificial skin survive to maintain hair shaft formation, it was shown that the hair matrix of the hair bulb portion and hair papilla cells are surviving in artificial skin having formed the epidermis after incorporating hair follicles into the dermal layer (Figures 3B and 3C), as well as in artificial skin having incorporated hair follicles after formation of dermal and epidermal layers (Figure 4B).

### 4-3. Hair shaft growth

Using a stereomicroscope (Stemi2000, Zeiss), macrophotographs were taken over time from the time of hair follicle incorporation (Day 0) until Days 3, 4, 7, 14, and the change in hair shaft length was measured with image analysis software (AxioVision, Zeiss and Image J, NIH). Moreover, hair follicle and hair shaft lengths before transplantation, as well as hair follicle and hair shaft lengths from hair follicles harvested over time from artificial skin that incorporated hair follicles were similarly measured by image analysis, and hair shaft growth was quantitatively functionally evaluated.

As a result, it was shown that in artificial skin having formed the epidermis after incorporating hair follicles into the dermal layer, hair shafts grow for 4 days from hair follicles incorporated in the artificial skin (Figure 5A-C). Further, it was shown that hair shaft growth is also maintained in artificial skin having incorporated hair follicles after formation of dermal and epidermal layers (Figure 6).

From these results, it was shown that the artificial skin obtained by the method of the present invention has functional skin appendage.

### Example 2: Production of artificial skin further having fat tissue layer

### 1. Production of 6-well format artificial skin

Production of 6-well format artificial skin was performed by the following procedures.

### <Cell culture>

Normal human neonatal preputium fibroblasts (HDFn) and normal human neonatal preputium epidermal keratinized cells (HEKn) were purchased as frozen cells after first passage from KURABO. HDFn and HEKn cells were rapidly thawed in a warm bath at 37°C, and then washed with 10% fetal bovine serum and Dulbecco modified Eagle's medium (DMEM 10) comprising 50 units/ml of penicillin and 50 µg/ml of streptomycin, seeded at a cell density of 2,500 cells/cm² in a culture plastic dish filled with DMEM 10 medium for HDFn cells and HuMedia-KG2 medium (KURABO) for HEKn cells, and these were called second passage cells. Passage culture was according to conventional means, digested with a solution of D-PBS (-) (Nacalai Tesque) supplemented with 0.25% Trypsin-1 mM EDTA (Invitrogen), dispersed, and then the cell density was adjusted and subcultured under medium conditions set for each cell. Normal human cells were subcultured to the second passage and subjected to artificial skin production, or set as frozen stocks and employed for artificial skin production after thawing as third passage cells. When used as frozen stocks, both were maintained in culture until 60 - 80% confluent, and used at a state of 80% or higher confluency for artificial skin production.

### <Production of fibroblast-containing collagen gel>

Subcultured HDFn cells were single-celled by digestion by trypsin EDTA solution. Subsequently, a neutral collagen solution comprising final concentrations of 3.8 mg/ml collagen (I-AC 5 mg/mL, KOKEN), 1 x DMEM (SIGMA), 10 mM NaHCO3 (Wako Pure Chemical Corporation), 10 mM HEPES buffer (titered and adjusted to pH 7.4, Wako Pure Chemical Corporation), and 5% fetal bovine serum was produced, and HDFn cells pelleted by centrifugation was dispersed while gently stirring at ice temperature condition. As an additive to neutralize an acidic collagen solution to obtain a cell survival environment, 10 x concentration DMEM, 1M NaHCO3, and 1M HEPES buffer were produced, and stored as stock under refrigeration at 4°C. HDFn cells dispersed in the neutral collagen solution were dispensed in a 6-well cell culture insert (0.4 µm/high density pore) at 400 µl each, left standing to gel under 37°C, 5% CO₂, and 100% humidity condition for 30 minutes to form an artificial dermal layer with a thickness of 1.0 - 1.5 mm. In order to make the surface of the fibroblast-containing collagen gel horizontal with the filter surface of the top of the cell culture insert, as well as to allow uniform gelling progress, preparation of the collagen solution and cell dispersion were performed on ice, and the prepared collagen solution was stored buried in ice until dispensed into the cell culture insert. The cell culture insert having formed an artificial dermal layer by gelling of the cell-containing collagen solution was set in a 6-well culture plate filled with DMEM 10 medium comprising 10 ng/ml of FGF2 (Wako Pure Chemical Corporation), and maintained in culture until epidermal layer formation. The following culture conditions were all 37°C, 5% CO2, and 100% humidity condition.

### <Epidermal layer formation>

The epidermal layer was formed on the surface of the fibroblast-containing collagen gel produced by the above method to produce artificial skin.

The specific method is as follows. HEKn cells proliferated to 60% - 80% confluency were single-celled by trypsin digestion, and suspended in HuMedia-KG2 medium to 3.7 x 10⁶ cells/ml. One milliliter of cell suspension per well was seeded overlaid on the fibroblast-containing collagen gel produced in (4) in Example 1 and cultured to form a keratinized epidermal layer on the fibroblast-containing collagen gel. The overlaid HEKn cells were cultured in HuMedia-KG2 medium or DMEM 10 medium until Day 4 after seeding, and total medium exchange was performed on Days 1, 2, and 3 after HEKn cell seeding with the same medium condition as the overlaid seeding. The operation described above was performed after replenishing the collagen gel following the shrinking of the fibroblast-containing collagen gel. The amount of DMEM 10 medium comprising 10 ng/ml of FGF2 in the well was adjusted so as to be the same with the liquid surface of the HuMedia-KG2 medium in the cell culture insert. By removing the medium in the cell culture insert on Day 4 after overlay of HEKn cells, air culture, which is a culture condition in which the epidermal layer is exposed to low oxygen partial pressure condition (under 37°C, 5% CO₂, 12.5% O₂, and 100% humidity condition) was initiated.

The above artificial skin after performing two days of air culture was overlaid on the subcutaneous fat-containing collagen gel (fat tissue layer) described below, and then subjected to hair follicle incorporation.

### 2. Production of subcutaneous fat-containing collagen gel (fat tissue layer)

Skin tissue having undesired regions such as connective tissue trimmed from mouse dorsal skin tissue was cut into ribbons, and then fat tissue was harvested with tweezers. The stripped fat tissue was dispersed in the neutral collagen solution prepared with the method according to (4) in Example 1, left standing to gel under 37°C, 5% CO₂, and 100% humidity condition for 30 minutes and further cut into 3 mm cubes, placed in a neutral collagen gel solution, and allowed to gel with a method similar to the above to have a subcutaneous fat layer. A collagen gel without fat tissue was also produced as control.

### 3. Preparation of hair follicles for transplantation (Figure 7)

The hair follicles employed in this Example were mouse whiskers prepared by the method described in "2. Production of artificial skin by forming epidermis after incorporating hair follicles into dermal layer" in Example 1 with the collagen sheath removed.

Mouse whiskers in anagen were harvested to produce intact hair follicles comprising collagen sheath (Figure 7 top row) and hair follicles with collagen sheath in the variable region removed (Figure 7 bottom row). Hair follicles for each condition were adhered to the bottom of a 6 cm plastic culture dish with a thin layer collagen gel, and subjected to immersion culture in DMEM/F12 (1:1) comprising 10% FBS under 5% CO2 environment until Day 3.

Stereomicroscopic follow-up was performed daily from the start of culture (Day 0). The arrows show hair shaft tips, and the arrowheads show the positions of the hair bulb portion. The right column shows magnified images of the hair bulb portion on culture Day 3. The dotted line shows the mesenchymal tissue boundary of the outermost layer of hair follicle. Ordinarily, in the structural change of a hair follicle from anagen via catagen to telogen, it is known that the hair bulb portion moves to the upper portion of the hair follicle to form a structure called a secondary hair bud. For this reason, the rising of the position of the hair bulb portion and the off-position of the mesenchymal tissue boundary of the hair follicle indicate that it is catagen or telogen.

Note that it is known that mouse whisker hair follicles are inside a living subject in a state wrapped in collagen sheath, and when an anagen hair follicle is subjected to ex vivo culture in a state attached to collagen sheath, hair shaft is elongated and anagen is maintained (e.g. Biochemical and Biophysical Research Communications 367 (2008) 299-304). On the other hand, it is well-known in the hair transplant medical care site that a hair follicle subjected to e.g. single hair follicle transplantation technology goes through catagen to enter telogen. Similarly, it was found that when mouse whisker hair follicles in anagen stimulated by external treatment such as removal of collagen sheath are subjected to ex vivo culture, off-position between the positions of the hair bulb portion and the mesenchymal tissue boundary of the hair bulb portion is produced, and transitions to catagen or telogen are made (Figure 7).

### 4. Production of artificial skin with subcutaneous fat and incorporation of hair follicles (Figure 8a)

Artificial skin after two days of air culture was placed on top of the fat tissue layer, and then the hair follicles were incorporated. Incorporation of hair follicles in the artificial skin was performed similarly to the method described in "2. Production of artificial skin by forming epidermis after incorporating hair follicles into dermal layer" of Example 1 by forming slits that penetrate both the epidermal layer and the fat tissue layer, producing hair follicles having the above collagen sheath removed, and incorporating them with tweezers. After incorporation of hair follicles, three days of organ culture was performed. The organ culture condition was DMEM/F12 (1:1) comprising 10% FBS, under 5% CO₂ environment, and carried out by semi-gas phase culture.

### 5. Comparison of hair follicle growth due to presence or absence of fat tissue layer (Figure 8b)

Figure 8b shows tissue images of transplanted hair follicles on organ culture Day 3. It was shown that hair follicles incorporated in artificial skin without fat tissue layer (Figure 8b left) had a linear hair follicle mesenchyme image (arrows) indicating catagen, whereas hair follicles incorporated in artificial skin with fat tissue layer (Figure 8b right) was anagen hair bulb portion. The dotted line in the right photograph shows the boundary between the dermis (Der) and fat tissue (Adp).

From the above results, it was shown that fat tissue has the effect of promoting hair follicle growth. In other words, it was shown that the artificial skin of the present invention could be evaluated for hair follicle growth by interaction between hair follicle and subcutaneous fat tissue.

### Example 3: Manufacture of artificial skin employing regenerated hair follicle primordiums

### 1. Induction of fat progenitor cells from REC cells

Fat progenitor cells were REC (Rapidly Expanding Cell, purchased from Bay bioscience Inc.) which is a commercially available mesenchymal lineage stem cell and induced according to prior literature (Mabuchi Y. et al., Stem Cell Reports, 1, 152-165, 2013).

REC was rapidly thawed in a warm bath at 37°C, and then washed with Adipogenic Maintenance Medium (Lonza), seeded in a culture plastic dish filled with the same medium at a cell density of 21,000 cells/cm², and cultured for three days. Next, in order to induce differentiation into fat cells, Adipogenic Maintenance Medium was removed and then substituted to Adipogenic Induction Medium (Lonza), and further cultured for 2 days. Collection of cells followed conventional means using a solution of D-PBS (-) (Nacalai Tesque) supplemented with 0.25% Trypsin-1 mM EDTA (Invitrogen). Expression of fat progenitor cell marker PPARγ gene in the cells obtained was confirmed, and these were employed as REC-derived fat progenitor cells.

### 2. Production of regenerated hair follicles for transplantation (Figure 9a)

From E18.5 mouse dorsal skin, skin epithelium and mesenchymal lineage cells single-celled by enzyme treatment were prepared, and these were employed to produce regenerated hair follicle primordiums. The production of regenerated hair follicle primordiums was performed with the methods described in WO2012/108069 and WO2012/115079.

The regenerated hair follicle primordiums were transferred to a cell culture insert, and subjected to seven days of organ culture with CMEM/F12 (1:1) medium comprising 10% FBS while adjusting the gas partial pressure with a multi-gas chamber. After seven days, emergence of hair follicles was confirmed under a stereomicroscope (Figure 9a, center left). These were divided according to each hair group (Figure 9a, center right) and subjected to incorporation in artificial skin.

### 3. Transplantation of regenerated hair follicles to artificial skin (Figure 9a)

The prepared regenerated hair follicles were incorporated in the following three types of artificial skin, subjected to three days of organ culture (DMEM/F12 (1:1) medium comprising 10% FBS, 5% CO2 environment), and then histological analysis was performed.

1. Epidermis + dermis model (artificial skin before hair follicle incorporation in Example 1)
2. Epidermis + dermis + fat tissue model (artificial skin before hair follicle incorporation in Example 2)
3. Epidermis + dermis + REC model (artificial skin of 1 having fat progenitor cells induced from REC incorporated in the transplantation pores that hair follicles are transplanted in)

In any artificial skin, a 25 G injection needle was employed to form a transplantation pore that reaches the lower layer of the artificial skin, and incorporation was performed so that the depth will match between the artificial skin surface and the position to be the hair pore portion of the divided hair follicle.

In the "epidermis + dermis + REC model," a micropipette was employed to inject 0.2 µl of REC-derived fat progenitor cell agglomerate with the culture medium removed into the transplantation pore, and then hair follicles were incorporated with a method similar to above.

### 4. Histological analysis of transplanted hair follicles (Figure 9b)

In Figure 9b, the top row shows the result of the "epidermis + dermis model," the middle row shows the "epidermis + dermis + fat tissue model," and the bottom row shows the "epidermis + dermis + REC-derived fat progenitor cell model." Magnification of the rectangular area shown in the left column of Figure 9b, the low-power magnified view, is shown in the right column. In any of the models, the epithelium tissue of the transplanted hair follicles is connected to the artificial skin epidermal layer.

The regenerated hair follicle tissue transplanted to the "epidermis + dermis model" was stray from the differentiation tendency of hair follicle tissue, and although mesenchymal cells wrapped in epithelium tissue (Figure 9b, top row right, arrow) were seen, hair matrix cell differentiation of epithelial cells was not seen.

In the "epidermis + dermis + fat tissue model," although numerous mesenchymal cell agglomerates enveloped by the epithelium was confirmed in the transplanted hair follicles (Figure 9b, middle row right, arrows), the result showed differentiation tendency specific to the hair bulb portion to be low in epithelial cells surrounding the mesenchymal cells. Moreover, although elongation of the epithelium tissue was seen in both models (* asterisks), differentiation tendency into hair follicle epithelium was not histologically confirmed.

In the hair follicles transplanted to the "epidermis + dermis + REC-derived fat progenitor cell model," mesenchymal cells were incorporated by the epithelium with a strong differentiation tendency into hair matrix (Figure 9b, bottom row, white arrow). Further, a hair shaft-like structure (HS) with a strong keratinization tendency was confirmed in the center of the epithelium of the transplanted hair follicles. REC-derived fat progenitor cells were distributed around the hair follicles (arrowheads) . As structures in artificial skin, Epi indicates the epidermal layer, Der indicates the dermal layer, and Adp indicates the subcutaneous fat tissue.

### Example 4: Effect of fat tissue and fat progenitor cells on hair shaft growth of hair follicles in artificial skin

Mouse whiskers in anagen were harvested to produce hair follicles with collagen sheath in the variable region removed. Said hair follicles were incorporated into the artificial skin of the "epidermis + dermis model (no fat tissue)," the "epidermis + dermis + fat tissue model," or the "epidermis + dermis + REC-derived fat progenitor cell (two or seven days of induction period) model" of Example 3, and subjected to organ culture for three days.

The organ culture condition was DMEM/F12 (1:1) comprising 10% FBS, under 5% CO2, 02 atmospheric partial pressure environment, and carried out by semi-gas phase culture. Hair shaft growth on culture Day 3 was macrophotographed with a stereomicroscope (Stemi2000, Zeiss), and the change in hair shaft length was measured with image analysis software (AxioVision, Zeiss and Image J, NIH). Moreover, hair follicle and hair shaft lengths before transplantation, as well as hair follicle and hair shaft lengths from hair follicles harvested over time from artificial skin that incorporated hair follicles were similarly measured by image analysis, and hair shaft growth was quantitatively functionally evaluated.

The result is shown in Figure 10. Compared to the "epidermis + dermis model," the "epidermis + dermis + fat tissue model" or the "epidermis + dermis + REC-derived fat progenitor cell model" showed hair shaft growth effect, and in particular, REC-derived progenitor cells with seven days of induction period showed the highest hair follicle growth effect.

From the above, it was shown that the hair follicles in the artificial skin manufactured by the method of the present invention showed similar behaviors to the hair follicles in the skin of a living subject. In other words, the artificial skin manufactured by the method of the present invention reproduced structures and functions similar to the skin of a living subject closer than a conventional artificial skin. Moreover, it was also shown that it is possible to introduce not only skin appendage such as hair follicles but also fat and similar cells in the artificial skin according to the present invention. Accordingly, it can be said that the artificial skin of the present invention has extremely high usefulness in e.g. drug effect and safety evaluation of pharmaceuticals and cosmetics against skin.

## Claims

1. A method for manufacturing artificial skin having hair follicles, sebaceous glands, and hair pores, **characterized in that** it comprises each of the following steps:
(A) : a step of preparing an artificial skin having dermal and epidermal layers or an artificial skin having only a dermal layer; and
(B): a step of transplanting isolated hair follicles to the artificial skin prepared in step (A);
wherein
said isolated hair follicle comprises a sebaceous gland, and
said isolated hair follicle is transplanted to said artificial skin so that the surface of said dermal layer of said artificial skin is in alignment with the position of the hair pore portion of said isolated hair follicle.

2. The method according to claim 1, **characterized in that** artificial skin having only a dermal layer is used in said step (A), and the method comprises the following step after said step (B):
(C): a step of forming an epidermal layer on said dermal layer of said artificial skin.

3. The method according to claim 1, **characterized in that** the dermal layer of the artificial skin in said step (A) is formed by gelling a mixed solution comprising fibroblasts, collagen, and culture medium.

4. The method according to claim 1, **characterized in that** the dermal layer of the artificial skin in said step (A) is formed by gelling a mixed solution comprising fibroblasts, collagen, and culture medium, and then further adding a mixed solution comprising collagen and culture medium, and allowing it to gel and repeating this at least once or more times.

5. The method according to claim 1, **characterized in that** the epidermal layer of the "artificial skin having dermal and epidermal layers" in said step (A) is formed by applying a mixed solution comprising keratinized cells and culture medium to the surface of said dermal layer, and subjecting them to culture.

6. The method according to claim 2, **characterized in that** said step (C) is a step of forming the epidermal layer by applying a mixed solution comprising keratinized cells and culture medium to the surface of said dermal layer of said artificial skin, and subjecting them to culture.

7. The method according to claim 1, **characterized in that** said step (A) is:
(A) : a step of preparing an artificial skin having a dermal layer, an epidermal layer, and a fat tissue layer.

8. The method according to claim 1, **characterized in that** it comprises the following step after said step (A) and before said step (B):
(A'): a step of embedding fat progenitor cells at the site of artificial skin that isolated hair follicles are transplanted in step (B).

9. The method according to any one of claims 1 to 8, **characterized in that** said isolated hair follicle is a hair follicle isolated from animal skin.

10. The method according to claim 9, **characterized in that** said animal is a human.

11. The method according to any one of claims 1 to 8, **characterized in that** said isolated hair follicle is an artificially induced regenerated hair follicle.

12. The method according to claim 11, **characterized in that** said regenerated hair follicle is a regenerated hair follicle manufactured by putting a first cell aggregate composed substantially of mesenchymal lineage cells and a second cell aggregate composed substantially of epithelial cells in close contact and culturing them inside a support.

13. The method according to claim 12, **characterized in that** at least either one of said mesenchymal lineage cells or said epithelial cells is obtained by inducing undifferentiated cells.

14. The method according to claim 12, **characterized in that** at least either one of said mesenchymal lineage cells or said epithelial cells is singled cells derived from animal hair follicles.

15. An artificial skin having hair follicles and sebaceous glands, **characterized in that** it further has hair pores.

16. The artificial skin according to claim 15, comprising:
a dermal layer consisting of a gel comprising fibroblasts, collagen, and culture medium,
an epidermal layer that is formed on the surface of said dermal layer and substantially consists of keratinized cells, and
a hair follicle having a sebaceous gland,
wherein
said hair follicle penetrates said epidermal layer and is buried into said dermal layer, and
the hair pore portion of said hair follicle is connected to said epidermal layer.

17. The artificial skin according to claim 16, **characterized in that** it further has a fat tissue layer in the lower part of said dermal layer.

18. The artificial skin according to claim 16, **characterized in that** said hair follicle is covered with fat progenitor cells in said dermal layer.

19. The artificial skin according to any one of claims 15 to 17, **characterized in that** said artificial skin is produced by the method according to any one of claims 1 to 14.
